# EUROPEAN PATENT SPECIFICATION

(11) **EP 0 596 130 B1**
(45) Date of publication and mention of the grant of the patent: **24.09.1997**
(21) Application number: 93904376.6
(22) Date of filing: 02.03.1993
(51) Int. Cl.: C07C 209/68, C07C 211/53, C07C 231/06, C07C 233/62

(54) **PRODUCTION OF 2-HALO-3,5-DIFLUOROANILINE, INTERMEDIATE FOR PRODUCING THE SAME, AND PRODUCTION OF SAID INTERMEDIATE**
VERFAHREN ZUR HERSTELLUNG VON 2-HALO-3,5-DIFLUORANILIN SOWIE ZWISCHENPRODUKT DAFÜR UND DESSEN HERSTELLUNG
PRODUCTION DE 2-HALO-3,5-DIFLUOROANILINE, INTERMEDIAIRE SERVANT A PRODUIRE CE COMPOSE, ET PRODUCTION DUDIT INTERMEDIAIRE

(30) Priority: 06.03.1992 JP 82997/92
(43) Date of publication of application: 11.05.1994
(73) Proprietor: SDS Biotech K.K., Minato-ku Tokyo 105 (JP)
(72) Inventor: MATSUSHITA, Utaro, Yokohama Works, Yokohama-shi, Kanagawa 221 (JP); WATANABE, Masao, Yokohama Works, Yokohama-shi, Kanagawa 221 (JP); TACHIBANA, Yoshiyuki, Yokohama Works, Yokohama-shi, Kanagawa 221 (JP)
(74) Representative: Strehl Schübel-Hopf Groening & Partner
(86) International application number: JP9300265
(87) International publication number: WO9317996

(56) References cited:
- EP-A- 0 415 595
- JP-A- 5 065 250

## Description

This invention relates to a method for the production of 2-halo-3,5-difluoroanilines, which are useful as raw materials or intermediate compounds for medicines, agricultural chemicals or industrial chemicals, intermediate compounds thereof and a method for the production of such intermediate compounds. More particularly, the present invention relates to a method for the production of 2-halo-3,5-difluoroanilines by hydrolyzing the nitrile group of a 4-amino-5-halo-2,6-difluoroisophthalonitrile in sulfuric acid to convert the diamide compound to a carboxylic acid derivative, and further subjecting it to decarboxylation, and novel 4-amino-5-halo-2,6-difluoroisophthalic acid diamide which are intermediate compounds for their production as well as production method therefor.

### BACKGROUND ART

2-Halo-3,5-difluoroanilines, objective compounds of the production method of the present invention, i.e., 2,3,5-trifluoroaniline and 2-chloro-3,5-difluoroaniline, are useful as intermediate compounds for the synthesis. For example, 2-chloro-3,5-difluoroaniline when reacted with hydrogen in the presence of a catalyst converts into 3,5-difluoroaniline with readily releasing the chlorine atom at the 2-position, and various useful compounds such as agricultural and horticultural bacteriocides can be synthesized from the 3,5-difluoroaniline.

As for the production methods for trihaloanilines, Finger et al. reported a method for the production of 2-chloro-3,5-difluoroanilines in which the amino group of 2,4-difluoro-6-nitroaniline is substituted with chlorine and then the nitro group thereof is substituted with an amino group (J. Fluorine Chem., 1, 415-425 (1971/1972)), and a method for the production of 2,3,5-trifluoroaniline by deaminating 2-nitro-3,4,6-trifluoroaniline and then reducing the nitro group thereof to an amino group (J. Am. Chem. Soc., 73, 152 (1951)).

In the above-described methods, after its amino group is acetylated, 2,4-difluoroaniline is nitrated, and then hydrolyzed to release the acetyl group to obtain 2,4-difluoro-6-nitroaniline, which is then subjected to Sandmeyer reaction in which the amino group is diazotated in the presence of cuprous chloride to synthesize 2-chloro-3,5-difluoronitrobenzene and 2,3,5-trifluoronitrobenzene, followed by further reduction of the nitro groups, respectively, to obtain 2-chloro-3,5-difluoroaniline and 2,3,5-trifluoroaniline.

The methods have various problems, for example, that raw materials are hardly available industrially, they require long reaction steps starting from 2,4-difluoroaniline as raw material, yield is low since isomers are by-produced, and it takes a lot of time to separate the by-produced isomers for purification.

On the other hand, as techniques relating to the present invention, respective reactions of hydrolyzing nitriles through amides to carboxylic acid derivatives, and decarboxylation reaction for carboxylic acid derivatives are known as unit reactions.

For example, taking the decarboxylation reaction for aminocarboxylic acids as an example, it is well known to produce aniline by decarboxylation of anthranilic acid. Also, as for decarboxylation of fluorinated aminocarboxylic acids, examples of synthesis of corresponding fluorinated anilines by decarboxylation of 2-fluoro-, 3-fluoro-, 3,4-difluoro-, or 2,3,4,5-tetrafluoroanthranilic acid are known (Dokl. Akad. Nauk. SSSR. 1968, 179(2), 356-357, Chem. Abst., 69: 76350j, ibid. 97: 197986h, EP55,629)

EP-A-415 595 relates to processes for the preparation of fluoroaromatics, inter alia 2,3,5-trihaloanilines, involving the hydrolysis of the respective dinitrile compounds in an acidic medium.

### OBJECT OF INVENTION

Therefore, it is an object of the present invention to provide an industrially advantageous method for producing 2-halo-3,5-difluoroanilines which are useful as raw materials for medicines, agricultural chemicals or industrial chemicals.

### DISCLOSURE OF INVENTION

As a result of intensive research, the present inventors have confirmed that when 4-amino-5-halo-2,6-difluoroisophthalonitriles, raw materials easily available industrially, are hydrolyzed in an aqueous sulfuric acid solution, 2-halo-3,5-difluoroanilines can be obtained in high yields in a step much shorter than conventionally, that 4-amino-5-halo-2,6-difluoroisophthalic acid diamides, novel compounds, can be isolated as intermediate compounds depending on the conditions, and that the objective compounds can be obtained more efficiently by changing the conditions of hydrolysis until the intermediate compounds are obtained and those of hydrolysis from the intermediate compounds to the objective compounds. The present invention is based on the discovery.

That is, the present invention provides:
1. 4-Amino-5-halo-2,6-difluoroisophthalic acid diamides represented by general formula (II) 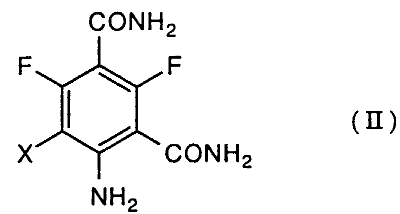 wherein X is fluorine or chlorine.
2. A production method for producing 4-amino-5-halo-2,6-difluoroisophthalic acid diamide represented by general formula (II) 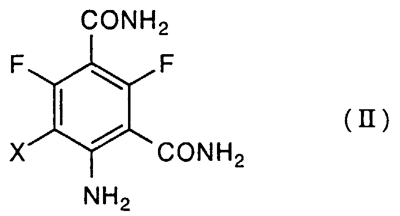 wherein X is fluorine or chlorine, comprising the steps of:
   heating 4-amino-5-halo-2,6-difluoroisophthalonitriles represented by general formula (I) 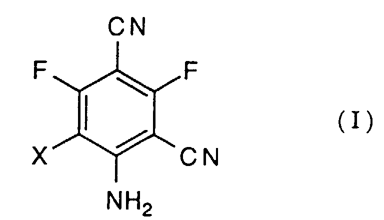 wherein X is fluorine or chlorine, in the presence of sulfuric acid in a concentration of not lower than 50% by weight and containing an at least double molar amount, relative to said 4-amino-5-halo-2,6-difluoroisophthalonitriles, of water at a temperature of from 80°C to a boiling point of reaction mixture for 5 minutes to 3 hours to have the nitrile groups to hydrolysis reaction,
   diluting the cooled reaction mixture with water, and
   collecting the precipitated crystals.
3. A production method as described in claim 2, wherein said heating takes place in the presence of sulfuric acid in a concentration of 80% to 98% by weight for 20 minutes to 1 hour.
4. A production method for producing 2-halo-3,5-difluoroanilines represented by general formula (III) 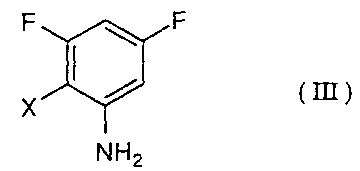 wherein X is fluorine or chlorine, comprising the step of:
   heating 4-amino-5-halo-2,6-difluoroisophthalic acid diamide represented by general formula (II) 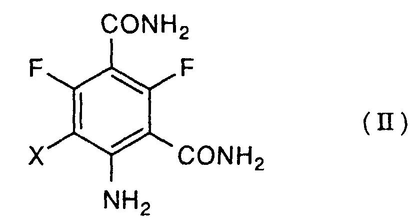 wherein X has the same meaning as defined above, in the presence of sulfuric acid in a concentration of not lower than 30% by weight at a temperature of from 80°C to a boiling point of reaction mixture.

Hereinafter, the methods of the present invention will be described in greater detail.

The compounds represented by general formula (I) used as raw materials in the method of the present invention, i.e., 4-amino-2,5,6-trifluoroisophthalonitrile corresponding to X being fluorine, and 4-amino-5-chloro-2,6-difluoroisophthalonitrile corresponding to X being chlorine are both known compounds and can be readily produced using as a raw material 2,4,5,6-tetrachloroisophthalonitrile which is produced on large scale industrially as crude preparation of an agricultural and horticultural bacteriocide (general name: chlorothalonil, trade name: Daconil (registered trademark)).

More particularly, 2,4,5,6-tetrachloroisophthalonitrile is subjected to halogen substitution in a system of dry particle potassium fluoride (KF)/dimethylformamide (DMF) to obtain 5-chloro-2,4,6-trifluoroisophthalonitrile. Similarly, halogen substitution of 2,4,5,6-tetrachloroisophthalonitrile is carried out in a system of dry particle potassium fluoride (KF)/sulfolane to obtain 2,4,5,6-tetrafluoroisophthalonitrile. Then, amination of the fluorine of each of the resulting compounds at the 4-position can produce 4-amino-5-chloro-2,6-difluoroisophthalonitrile and 4-amino-2,5,6-trifluoroisophthalonitrile with ease (Bulletin of Japan Chemical Society 1985 (11), 2155-2160, Chem. Abst. 104: 202306f, ibid. 105: 152649t, DE3530941).

According to the method of the present invention, 4-amino-5-halo-2,6-difluoroisophthalonitriles of general formula (I) above are heated in the presence of sulfuric acid so that two nitrile groups can be hydrolyzed to carboxamide to prepare the compounds of general formula (II) above, and then the carboxamide groups are hydrolyzed to give carboxylic acid derivatives, followed by decarboxylation of the carboxylic acids to finally derive 2-halo-3,5-difluoroanilines.

While hydrolysis of nitrile groups to carboxamide groups, hydrolysis of carboxamide groups to carboxyl groups, and decarboxylation of carboxyl groups are known as unit reactions as described in the BACKGROUND ART above, it is difficult to estimate if respective specific compounds could be derived finally by decarboxylation reaction. For example, the compound of general formula (I) above in which all the halogen atoms correspond to chlorine atoms, i.e., 4-amino-2,5,6-trichloroisophthalonitrile can give rise to dicarboxamide in high yields. However, it is very difficult to produce trichloroaniline from dicarboxamide through a carboxylic acid derivative. Also, in the hydrolysis path of the present invention, the reaction rate differs greatly depending on the kind and concentration of acids used such that the hydrolysis reaction will not proceed when there is used hydrochloric acid, for example. Therefore, selection of reaction conditions (especially, concentration of sulfuric acid and reaction temperature) is important for efficiently obtaining the objective compounds.

In the present invention, the hydrolysis reaction proceeds in two steps through the intermediate compounds of general formula (II), which are novel compounds. However, it is unnecessary to separate the intermediate compounds of general formula (II) from the viewpoint of reaction operations but the objective compounds may be produced from the raw material compounds of general formula (I) and sulfuric acid in a single reaction vessel (in one batch) by a continuous operation. For the purpose of comparison, 4-amino-5-halo-2,6-difluoroisophthalonitriles represented by general formula (I) are heated in sulfuric acid containing at least 4 times molar amount of water relative to the raw material dinitrile compound at a temperature of from 80°C to a boiling point of reaction mixture so that hydrolysis and decarboxylation reactions can be performed to obtain 2-halo-3,5-difluoroanilines. In this case, it is preferred to use sulfuric acid in a concentration of not lower than 50% by weight, particularly not lower than 60% by weight, since the reaction takes a long time under conditions where the concentration of sulfuric acid is not higher than about 50% by weight. After the reaction is over, the reaction mixture may be cooled in a conventional manner, and after it is diluted with water, it may be extracted with a solvent to separate the objective compound, which may be further purified by recrystallization.

The present inventors further investigated on reaction conditions, and as a result they have confirmed that it is particularly effective to carry out the hydrolysis reaction of from general formula (I) to the intermediate compound of general formula (II) in sulfuric acid in a higher concentration, and the hydrolysis and decarboxylation reactions of from the intermediate compound of general formula (II) the objective compound through the carboxylic acid derivative in sulfuric acid in a lower concentration with increasing the amount of water as compared with the preceding step.

More particularly, in the hydrolysis reaction in the first step, sulfuric acid in a concentration of at least 50% by weight, preferably 80 to 98% by weight, since the reaction takes a long time and practically sufficient yields cannot be obtained if the concentration of sulfuric acid is lower.

As for the amount of water in the hydrolysis reaction in the first step, at least 2 times the stoichiometric amount, i.e., 2 moles of water per mole of the raw material compound of general formula (I) is sufficient. If it exceeds 2 moles, hydrolysis proceeds, resulting in decreased yields of the intermediate compounds of general formula (II).

Note that the amount of sulfuric acid used relative to the charged raw materials depends on the concentration of sulfuric acid and the amount of water.

While reaction temperature is not limited strictly, it is necessary to perform the reaction at a temperature of from 80°C to a boiling point of reaction mixture since at low temperature, the reaction takes a long time as in the case of the concentration of sulfuric acid, and practically there can be obtained no sufficient yields. Preferred reaction temperature is 90 to 150°C, and particularly preferred temperature in practice is 100 to 120°C.

The reaction is carried out usually at atmospheric pressure. However, it may be carried out under reduced pressure or under pressure as necessary.

The reaction time cannot be defined generally since it may differ depending on other reaction conditions, particularly the concentration of sulfuric acid and reaction temperature. Under optimal conditions, the objective compound of general formula (II) can be obtained almost quantitatively in about 5 to 30 minutes. However, it is sufficient for the reaction to proceed for at most 3 hours so far as the concentration of sulfuric acid and reaction temperature are within the above-described ranges. Practical range is from 5 minutes to 3 hours, and usually a range of about 20 minutes to 1 hour is appropriate.

Usually, it is desirable to carry out the reaction in the first step with stirring since the raw material compounds of general formula (I) are insoluble in sulfuric acid and initially the reaction mixture is non-uniform. According as the compounds of general formula (II) are produced while the reaction proceeds, a uniform reaction mixture is obtained in a relatively short time.

No solvent has to be used, and the compounds of general formula (II) can be obtained almost quantitatively corresponding to the raw materials used by simply mixing the raw material compounds with sulfuric acid to allow reaction at a predetermined temperature with stirring. However, the use of solvents is optional in order to carry out the reaction in a uniform system from its initial stages. In this case, any solvent may be used that can dissolve the raw material compounds well and is stable under the reaction conditions.

The intermediate compounds of general formula (II) may be isolated from the reaction mixture for purification as necessary. For example, after it is cooled, the reaction mixture may be diluted with water to precipitate crystals, which may be collected by filtration, washed, dried and purified by recrystallization.

The compounds of general formula (II), i.e., 4-amino-2,5,6-trifluoroisophthalic acid diamide and 4-amino-5-chloro-2,6-difluoroisophthalic acid diamide, are novel compounds, respectively, and their description and physical properties are as shown in examples hereinbelow.

The hydrolysis reaction in the second step may be performed using the compounds of general formula (II) isolated. However, since the compounds of general formula (II) are obtained almost quantitatively in the hydrolysis reaction in the first step, it does not have to separate the intermediate compounds from the reaction mixture and they can be used as they are in the hydrolysis and decarboxylation reactions in the second step successively. Note that it is preferred to add water to control the concentration of sulfuric acid without separating the intermediate compounds (II) from the reaction mixture in the first step since the reaction tends to proceed at low rates in the hydrolysis of diamides if the amount of water is small.

While the sulfuric acid concentration condition in the hydrolysis reaction in the second step does not have to be strict, it is suitable to use it usually not lower than 30% by weight, preferably 50 to 70% by weight. Although low concentration does not influence the reaction so greatly, the addition of water in a stage where the reaction switches over to the hydrolysis reaction in the second step to adjust the concentration of sulfuric acid to a low level causes a problem of increase in the amount of the dilution liquid since the concentration of sulfuric acid in the first reaction is relatively high as described above.

On the other hand, when the amount of water is small, there is a disadvantage of prolonged reaction time. Theoretically, the amount of water is 2 moles per mole of the intermediate compounds (II) as in the case of the reaction in the first step. Therefore, at least 2 moles of water is necessary. However, in order to obtain practically sufficient yields, it is desirable to use water in excess of the theoretical amount. While the amount of water needs not be controlled precisely, it is suitable to use usually about 2 to 140 times, preferably about 5 to 50 times, the theoretical amount. The amount of sulfuric acid to be used depends on the amounts of the compounds of general formula (II) as raw materials, the concentration of sulfuric acid, and the amount of water as in the case of the first step.

There is posed no strict limitation on the reaction temperature. However, it is desirable that the reaction is carried out at a temperature of from 80°C to a boiling point, preferably no lower than 110°C, since lower temperatures result in longer reaction time and in addition, no practically sufficient yield can be obtained. The reaction is carried out usually at atmospheric pressure. However, it may be performed under reduced pressure or under pressure as necessary. The reaction time cannot be defined generally since it may vary depending on other reaction conditions such as the concentration of sulfuric acid and reaction temperature. Usually, it is from about 30 minutes to 10 hours.

Separation and purification of the reaction products may be performed by a conventional method. For example, they can be separated from the reaction mixture by extraction with an aromatic solvent such as benzene, toluene, xylene, ethylbenzene or chlorobenzene and purified by recrystallization as necessary.

### BEST MODE FOR CARRYING OUT THE INVENTION

Hereinafter, the present invention will be described more concretely by reference examples, comparative examples and examples, which are mere examples, and the present invention should not be construed as being limited thereto. The scope of the present invention is defined by appended claims but not limited by the description which follows.

¹H-NMR and ¹³C-NMR were measured using tetramethylsilane as internal standard substance (0 ppm), and ¹⁹F-NMR was measured using trifluoroacetic acid as external standard substance (-76.5 ppm).

All percentages (%) except yield (%) in the following examples are by weight unless otherwise stated specifically.

### Reference Example 1: Preparation of 4-Amino-5-chloro-2,6-difluoroisophthalonitrile

25% Ammoniac water (70 g) was added to 43.3 g (0.2 mole) of 5-chloro-2,4,6-trifluoroisophthalonitrile. To this was added 1 ml of acetonitrile, and the mixture was stirred for 1 hour while cooling the reaction mixture with water to keep the reaction temperature to 20 to 25°C. Water (500 ml) was added to the reaction mixture, and crystals precipitated were filtered by aspiration and air-dried.
Yield: 46.1 g (water 6%)
IR (cm⁻¹): 3400, 3330, 3235, 2230, 1645, 1625, 1500, 1475, 1270, 1105, 830;
¹⁹F-NMR (CDCl₃): -94.12 ppm (d,1F), -96.67 ppm (d,1F).

### Reference Example 2: Preparation of 4-Amino-2,5,6-trifluoroisophthalonitrile

25% Ammoniac water (70 g) was added to 40 g (0.2 mole) of 2,4,5,6-tetrafluoroisophthalonitrile. To this was added 1 ml of acetonitrile, and the mixture was stirred for 1 hour while cooling the reaction mixture with water to keep the reaction temperature to 20 to 25°C. Water (500 ml) was added to the reaction mixture, and crystals precipitated were filtered by aspiration and air-dried.
Yield: 36 g
IR (cm⁻¹): 3395, 3330, 3235, 2280, 1680, 1615, 1555, 1520, 1370, 1330, 1175, 970, 920;
¹⁹F-NMR (CDCl₃): -100.25 ppm (d,1F), -119.75 ppm (d,1F), -158.83 ppm (q,1F).

### Comparative Example 1: Preparation of 2-chloro-3,5-difluoroaniline from 4-amino-5-chloro-2,6-difluoroisophthalonitrile

A flask was charged with 10.7 g (0.05 mole) of 4-amino-5-chloro-2,6-difluoroisophthalonitrile, to which was added 91.5% conc. sulfuric acid containing 3.6 g (0.2 mole) of water. After stirring well, the flask was heated on an oil bath with keeping the internal temperature at 115°C to dissolve the mixture. After it was stabilized, the temperature was further elevated to 140°C at which it was kept and the reaction was continued for 11 hours. After cooling down to 50°C, 40 ml of water was added, and the reaction mixture was extracted with 50 ml of toluene. Further, the extraction with 50 ml of toluene was repeated additional three times. The toluene solutions separated were joined, washed with 10 ml of water, and dried over anhydrous sodium sulfate. After solids were filtered off, the toluene was evaporated off under reduced pressure to obtain a pale yellow liquid, which was then analyzed quantitatively by HPLC. The amount of 2-chloro-3,5-difluoroaniline was 4.9 g (yield: 59.9%).

The pale yellow liquid thus obtained had the following infrared absorption spectrum and NMR spectrum, which coincided with the spectral data of standard 2-chloro-3,5-difluoroaniline.
IR (cm⁻¹): 3490, 3380, 1635, 1585, 1205, 1135, 985, 820;
¹H-NMR (CDCl₃): 4.26 ppm (br), 6.27 ppm (m);
¹⁹F-NMR (CDCl₃): -110.28 ppm (m,1F), -110.93 ppm (m,lF)

The acetyl derivative obtained after acetylation had a melting point of 86.5°C, which coincided with the reported value in the literature (86.5 to 86.7°C).

### Comparative Examples 2 to 7

Synthesis of 2-chloro-3,5-difluoroaniline was performed similarly to Comparative Example 1 by reacting 0.05 mole (10.7 g) of 4-amino-5-chloro-2,6-difluoroisophthalonitrile in sulfuric acid in a predetermined concentration and containing a predetermined amount of water as shown in Table 1 below. Table 1 shows reaction temperatures, reaction times and amounts (yields) of the objective compound together with the results of Comparative Example 1.

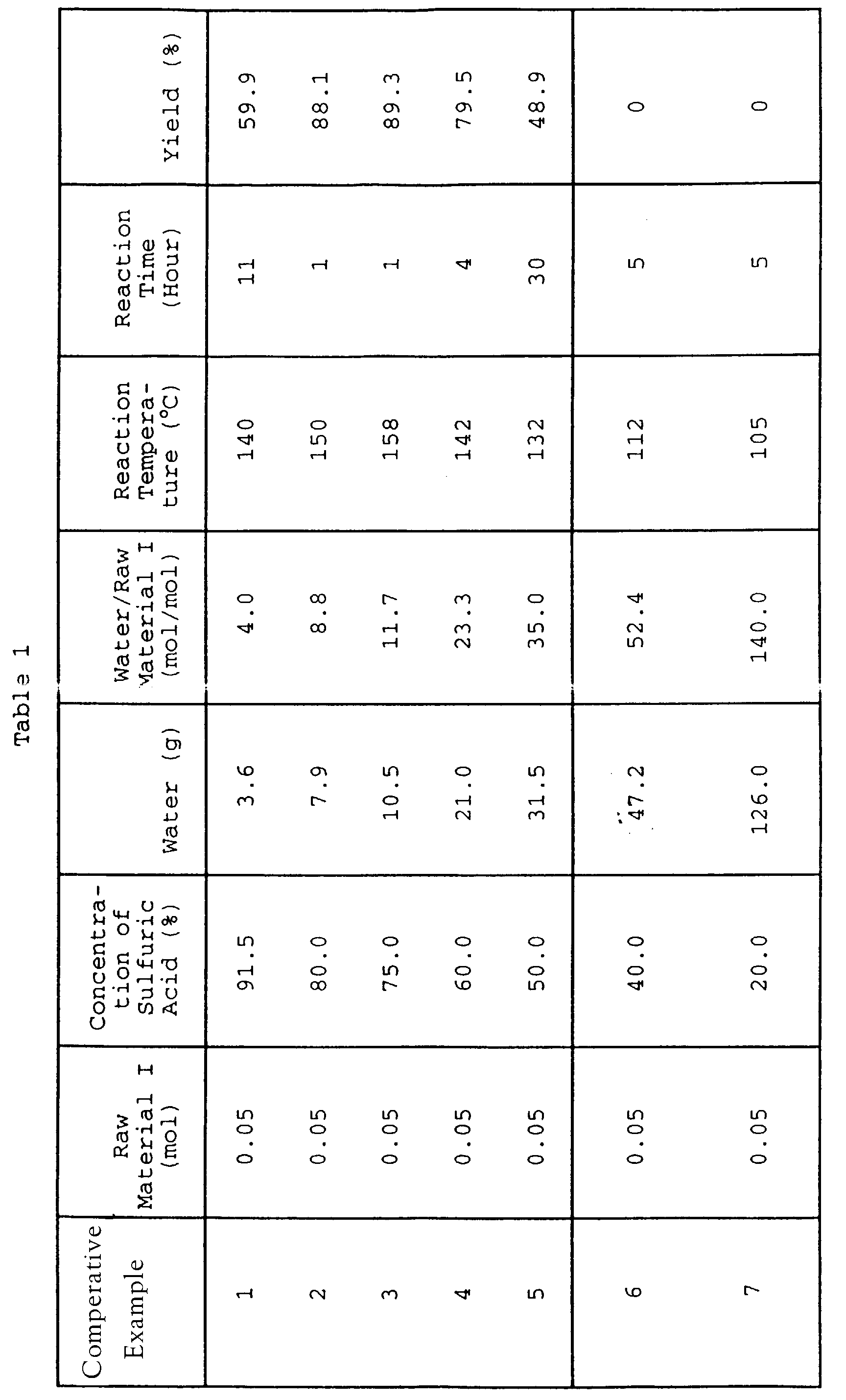

From the results shown in Table 1, can be seen that when 50% sulfuric acid was used (Comparative Example 5), the reaction took a long time, and the yield of 2-chloro-3,5-difluoroaniline was only 48.9% even after 30 hours' reaction while when 40% or less sulfuric acid was used (Comparative Examples 6 and 7) almost no 2-chloro-3,5-difluoroaniline was obtained after 5 hours' reaction. That is, it revealed that when the reaction was performed with sulfuric acid in a concentration of not higher than 50%, reaction times of no longer than 30 hours resulted in low yields of 2-chloro-3,5-difluoroaniline since hydrolysis rate of nitrile is low.

Comparative Example 1 was performed using water in the smallest theoretical amount, i.e., 4 moles per mole of 4-amino-5-chloro-2,6-difluoroisophthalonitrile. Under the conditions. the hydrolysis reaction proceeds rapidly up until 4-amino-5-chloro-2,6-difluoroisophthalic acid diamide but it proceeds slowly to further derivatives, and the yield of 2-chloro-3,5-difluoroaniline was 59.9% after 11 hours' reaction. The results in Table 1 suggest that the reaction can proceed more efficiently by switching over reaction conditions, particularly the concentration of sulfuric acid between the early and late stages of the hydrolysis step.

### Comparative Example 8: Hydrolysis in hydrochloric acid

A 300 ml three-necked flask was charged with 21.65 g (0.1 mole) of 4-amino-5-chloro-2,6-difluoroisophthalonitrile, to which was added 108.25 g of 20% conc. hydrochloric acid containing 86.6 g (4.81 moles) of water, and the mixture was heated under reflux on an oil bath. Heating at 108°C for 5 hours with stirring resulted in no hydrolysis reaction. HPLC analysis of the reaction mixture showed that no less than 99% of 4-amino-5-chloro-2,6-difluoroisophthalonitrile (raw material) remained unused.

Comparative Example 8 revealed that hydrolysis was very difficult to occur when hydrochloric acid was used instead of sulfuric acid.

### Example 1: Preparation of 2,3,5-trifluoroaniline by a 1 batch-2 stage method

A mixture of 9.85 g (0.05 mole) of 4-amino-2,5,6-trifluoroisophthalonitrile and 40 g of sulfuric acid in a concentration of 95.5% and containing 1.8 g (0.1 mole) of water was stirred for 30 minutes at 100 to 110°C. While keeping the temperature of the reaction mixture at no higher than 100°C, 21.8 g (1.21 moles) of water was added dropwise to adjust the concentration of sulfuric acid to 63.7%, and the mixture was stirred for 1 hour while heating under reflux. After cooling it to 50°C, the reaction mixture was extracted with 50 ml of toluene. Further, the extraction with 50 ml of toluene was repeated additional three times. The toluene layer was separated and dried over anhydrous sodium sulfate, followed by filtering solids. Evaporation of the toluene under reduced pressure afforded a pale yellow liquid of 2,3,5-trifluoroaniline.
Amount 6.6 g (yield 90.7%)

The pale yellow liquid thus obtained had the following infrared absorption spectrum and NMR spectrum, which coincided with the spectral data of standard 2,3,5-trifluoroaniline.
IR (cm⁻¹): 3480, 3370, 1640, 1510, 1220, 1115, 1100, 970, 815, 760;
¹H-NMR (CDCl₃): 3.96 ppm (br), 6.25 ppm (m);
¹⁹F-NMR (DMSO-d₆): -115.15 ppm (m,lF), -134.55 ppm (m,lF), -165.09 ppm (m,1F)

After decoupling of proton: -115.15 ppm (q,lF), -134.55 ppm (q,1F), -165.09 ppm (q,1F)

### Example 2: Preparation of 2-chloro-3,5-difluoroaniline by a 1 batch-2 stage method

A mixture of 64.05 g (0.3 mole) of 4-amino-5-chloro-2,6-difluoroisophthalonitrile and 240 g of sulfuric acid in a concentration of 95.5% and containing 10.8 g (0.6 mole) of water was stirred for 30 minutes at 100 to 110°C. While keeping the temperature of the reaction mixture at no higher than 100°C, 130.8 g (7.26 moles) of water was added dropwise to adjust the concentration of sulfuric acid to 63.7%, and the mixture was stirred for 1 hour while heating under reflux. After cooling it to 50°C, the reaction mixture was extracted with 300 ml of toluene. Further, the extraction with 300 ml of toluene was repeated additional three times. The separated toluene solutions were joined and washed with 50 ml of water, and dried over anhydrous sodium sulfate, followed by filtering solids. Evaporation of the toluene under reduced pressure afforded a pale yellow liquid of 2-chloro-3,5-difluoroaniline.
Amount 45 g (yield 90.9%)

### Example 3: Preparation of 4-amino-2,5,6-trifluoroisophthalic acid diamide

A flask was charged with 15 g (0.076 mole) of 4-amino-2,5,6-trifluoroisophthalonitrile, to which was added 46.4 g of 94% conc. sulfuric acid containing 2.78 g (0.154 mole) of water. After stirring well, the flask was heated on an oil bath with keeping the internal temperature at 95 to 100°C. After the mixture was dissolved and the temperature was stabilized, the reaction mixture was further heated at 120°C for 20 minutes. Thereafter, the reaction mixture was cooled down to 60°C, and poured in 200 ml of ice water. The mixture was stirred well to precipitate crystals, which were collected by filtration. To the crystals thus obtained was added 200 ml of water, and then sulfuric acid if any was neutralized with 10% aqueous sodium hydroxide solution. Thereafter, the crystals were filtered, air-dried to obtain pale yellow crude crystals of 4-amino-2,5,6-trifluoroisophthalic acid diamide (amount: 16.36 g, yield: 92.4%), which were recrystallized from acetonitrile and water.
Properties: White crystal
IR: 3365, 3185, 1655, 1465, 1415, 1365, 1255, 1135, 1090, 925, 805, 760, 685 cm⁻¹;
¹³C-NMR (DMSO-d₆): 102.3 ppm (dd), 103.8 ppm (d), 135.0 ppm (dd), 139.5 ppm (m), 147.5 ppm (dt), 152.9 ppm (dd), 160.7 ppm (s), 164.8 ppm (s);
¹⁹F-NMR (DMSO-d₆): -114.9 ppm (d), -135.7 ppm (d), -162.7 ppm (dd)

### Example 4: Preparation of 4-amino-5-chloro-2,6-difluoroisophthalic acid diamide

A flask was charged with 21.35 g (0.1 mole) of 4-amino-5-chloro-2,6-difluoroisophthalonitrile, to which was added 42.4 g of 91.5% conc. sulfuric acid containing 3.6 g (0.2 mole) of water. After stirring well, the flask was heated on an oil bath with keeping the internal temperature at 95 to 100°C. After the mixture was dissolved and the temperature was stabilized, the reaction mixture was further heated at 110°C for 20 minutes. Thereafter, the reaction mixture was cooled down to 60°C, and poured in 150 ml of ice water. The mixture was stirred well to precipitate crystals, which were collected by filtration. To the crystals thus obtained was added 100 ml of water, and then sulfuric acid if any was neutralized with 10% aqueous sodium hydroxide solution. Thereafter, the crystals were filtered, air-dried to obtain pale yellow crude crystals of 4-amino-5-chloro-2,6-difluoroisophthalic acid diamide (amount: 21.5 g, yield: 86.2%), which were recrystallized from acetonitrile and water.
Properties: White crystal
IR (cm⁻¹): 3345, 3205, 1655, 1620, 1355, 1120, 1095, 825, 795, 720;
¹³C-NMR (DMSO-d₆): 100.6 ppm (dd), 103.0 ppm (m), 146.5 ppm (m), 155.4 ppm (dd), 156.0 ppm (dd), 160.8 ppm (s), 164.9 ppm (s);
¹⁹F-NMR (DMSO-d₆): -111.0 ppm (d)

### Example 5: Preparation of 2,3,5-trifluoroaniline from 4-amino-2,5,6-trifluoroisophthalic acid diamide

To 23.3 g (0.1 mole) of 4-amino-2,5,6-trifluoroisophthalic acid diamide was added 150 g of 60% conc. sulfuric acid containing 60 g (3.33 moles) of water. After heating under reflux for 5 hours, the mixture was cooled down to room temperature, and 120 g of water was added thereto. Then, 100 ml of toluene was added thereto and extraction was performed at 90°C for 30 minutes. Further, the extraction with 100 ml of toluene was repeated additional three times, and the separated toluene solutions were joined together, and washed with 15 ml of water. Thereafter, the joined solution was dried over anhydrous sodium sulfate, and after filtration of solids, the toluene was evaporated under reduced pressure to obtain a pale yellow liquid.
Amount: 12.8 g (yield 87.1%)

### Example 6: Preparation of 2-chloro-3,5-difluoroaniline from 4-amino-5-chloro-2,6-difluoroisophthalic acid diamide

To 24.95 g (0.1 mole) of 4-amino-5-chloro-2,6-difluoroisophthalic acid diamide was added 150 g of 60% conc. sulfuric acid containing 60 g (3.33 moles) of water. The mixture was heated under reflux for 5 hours. Then, the mixture was cooled down to room temperature, and 120 g of water was added thereto. Then, 100 ml of toluene was added thereto and extraction was performed at 90°C for 30 minutes. Further, the extraction with 100 ml of toluene was repeated additional three times, and the separated toluene solutions were joined together, and washed with 15 ml of water. Thereafter, the joined solution was dried over anhydrous sodium sulfate, and after filtration of solids, the toluene was evaporated under reduced pressure to obtain a pale yellow liquid. The pale yellow liquid had infrared absorption spectrum and NMR spectrum which coincided with the spectral data of standard 2-chloro-3,5-difluoroaniline. Amount: 14.5 g (yield 88.7%)

### Example 7: Hydrolysis of 4-amino-5-chloro-2,6-difluoroisophthalic acid diamide

A 100 ml three-necked flask was charged with 2.5 g (0.01 mole) of 4-amino-5-chloro-2,6-difluoroisophthalic acid diamide, to which was added 21.0 g of 30% conc. sulfuric acid containing 14.7 g (0.82 mole) of water. The mixture was heated at 108°C for 20 hours under reflux. After the reaction was over, the reaction mixture was cooled to not higher than 60°C. Then, 20 ml of toluene was added thereto, and extraction was performed at 80 to 90°C for 1 hour. Further, the extraction with 20 ml of toluene was repeated twice at room temperature. The separated toluene solutions were joined together, and washed with 3 ml of 2% sodium hydrogen carbonate. Thereafter, the solution was dried over anhydrous sodium sulfate. After filtration of solids, the toluene was evaporated under reduced pressure to obtain a pale yellow liquid of 2-chloro-3,5-difluoroaniline.
Amount: 1.4 g (yield 85.6%)

### INDUSTRIAL APPLICABILITY

The present invention provides a production method which can give 2-halo-3,5-difluoroanilines using as raw materials 4-amino-5-halo-2,6-difluoroisophthalonitriles, which are easily available industrially, by simple operation of heating them in an aqueous sulfuric acid solution to effect hydrolysis and decarboxylation reactions, and novel 4-amino-5-halo-2,6-difluoroisophthalic acid diamides, intermediate compounds thereof and production method therefor.

2-Halo-3,5-difluoroanilines obtained according to the method of the present invention and 4-amino-5-halo-2,6-difluoroisophthalic acid diamides of the present invention are useful as raw materials or intermediate compounds for medicines, agricultural chemicals and industrial chemicals.

## Claims

1. 4-Amino-5-halo-2,6-difluoroisophthalic acid diamides represented by general formula (II) 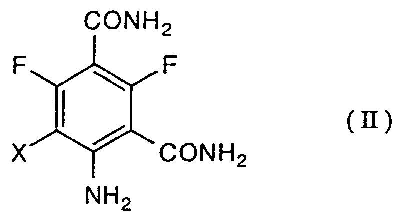 wherein X is fluorine or chlorine.

2. A production method for producing 4-amino-5-halo-2,6-difluoroisophthalic acid diamide represented by general formula (II) 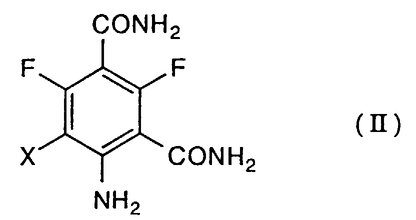 wherein X is fluorine or chlorine, comprising the steps of:
heating 4-amino-5-halo-2,6-difluoroisophthalonitriles represented by general formula (I) 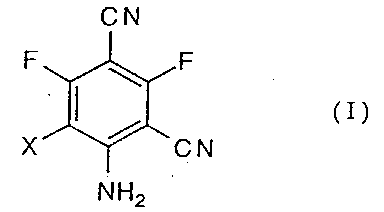 wherein X is fluorine or chlorine, in the presence of sulfuric acid of a concentration of not lower than 50% by weight and containing an at least double molar amount, relative to said 4-amino-5-halo-2,6-difluoroisophthalonitriles, of water at a temperature of from 80°C to a boiling point of reaction mixture for 5 minutes to 3 hours to have the nitrile groups subjected to a hydrolysis reaction,
diluting the cooled reaction mixture with water, and
collecting the precipitated crystals.

3. A production method as described in claim 2, wherein said heating takes place in the presence of sulfuric acid of a concentration of 80% to 98% by weight for 20 minutes to 1 hour.

4. A production method for producing 2-halo-3,5-difluoroanilines represented by general formula (III) 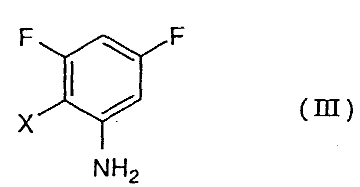 wherein X is fluorine or chlorine, comprising the step of:
heating 4-amino-5-halo-2,6-difluoroisophthalic acid diamide represented by general formula (II) 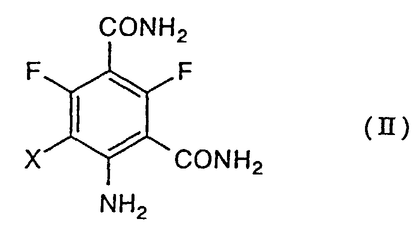 wherein X has the same meaning as defined above, in the presence of sulfuric acid of a concentration of not lower than 30% by weight at a temperature of from 80°C to a boiling point of reaction mixture.

## Patentansprüche

1. 4-Amino-5-halogen-2,6-difluorisophthalsäurediamide, dargestellt durch die allgemeine Formel (II) 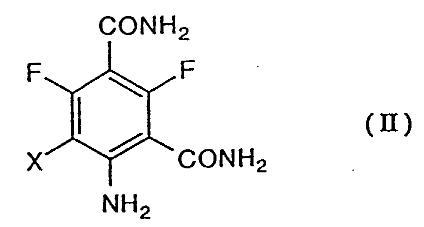 worin X Fluor oder Chlor bedeutet.

2. Verfahren zur Herstellung eines 4-Amino-5-halogen-2,6-difluorisophthalsäurediamids, dargestellt durch die allgemeine Formel (II) 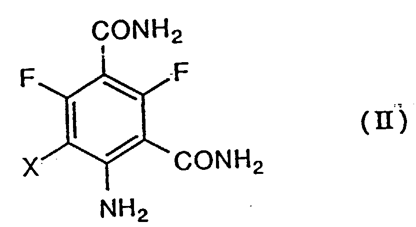 worin X Fluor oder Chlor bedeutet, welches folgende Stufen umfaßt:
Erhitzen von 4-Amino-5-halogen-2,6-difluorisophthalsäurenitrilen, die durch die allgemeine Formel (I) dargestellt sind 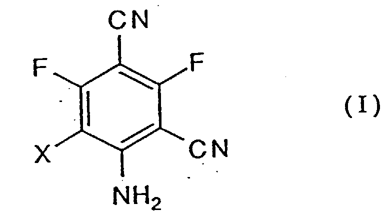 worin X Fluor oder Chlor bedeutet, in Gegenwart von Schwefelsäure einer Konzentration von nicht weniger als 50 Gew.-%, die mindestens die doppelte molare Menge an Wasser, bezogen auf die 4-Amino-5-halogen-2,6-difluorisophthalsäurenitrile enthält, auf eine Temperatur von 80°C bis zum Siedepunkt des Reaktionsgemisches während 5 Minuten bis 3 Stunden, wobei die Nitrilgruppen einer Hydrolysereaktion unterworfen werden,
Verdünnen des abgekühlten Reaktionsgemisches mit Wasser und
Gewinnen der abgeschiedenen Kristalle.

3. Verfahren gemäß Anspruch 2, wobei das Erhitzen in-Gegenwart von Schwefelsäure einer Konzentration von 80 bis 98 Gew.-% während 20 Minuten bis 1 Stunde stattfindet.

4. Verfahren zur Herstellung von 2-Halogen-3,5-Difluoranilinen, die durch die allgemeine Formel (III) dargestellt sind 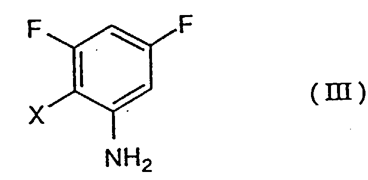 worin X Fluor oder Chlor bedeutet, welches folgende Stufe umfaßt:
Erhitzen von 4-Amino-5-halogen-2,6-difluorisophthalsäurediamid, dargestellt durch die allgemeine Formel (II) 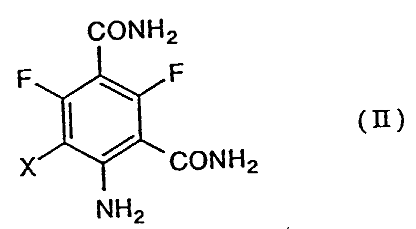 worin X die vorstehend definierte Bedeutung hat, in Gegenwart von Schwefelsäure einer Konzentration von nicht weniger als 30 Gew.-% bei einer Temperatur von 80°C bis zum Siedepunkt des Reaktionsgemisches.

## Revendications

1. 4-Amino-5-halo-2,6-difluoroisophtalyldiamides représentés par la formule générale (II) 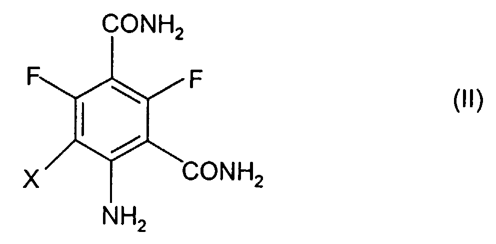 où X est le fluor ou le chlore.

2. Procédé de production pour produire le 4-amino-5-halo-2,6-difluoroisophtalyldiamide représenté par la formule générale (II) 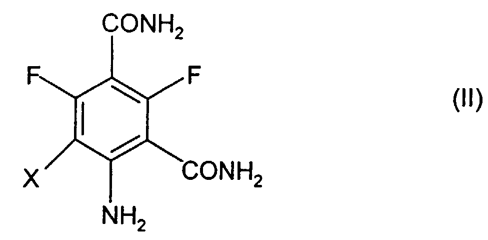 où X est fluor ou le chlore, comprenant les étapes de :
chauffer les 4-amino-5-halo-2,6-difluoroisophtalonitriles représentés par la formule générale (I) 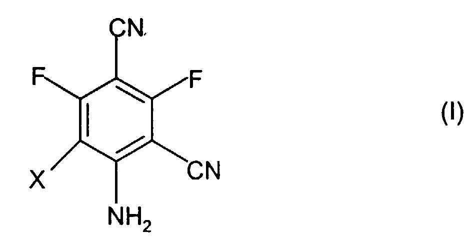 où X est le fluor ou le chlore, en présence d'acide sulfurique à une concentration qui n'est pas inférieure à 50 % en poids et contenant au moins une quantité molaire double par rapport auxdits 4-amino-5-halo-2,6-difluoroisophtalonitriles, d'eau à une température comprise entre 80°C et un point d'ébullition du mélange réactionnel pendant 5 min à 3 h pour provoquer la réaction d'hydrolyse des groupes nitrile,
diluer le mélange réactionnel refroidi avec de l'eau, et
recueillir les cristaux précipités.

3. Procédé de production selon la revendication 2, où ledit chauffage a lieu en présence d'acide sulfurique à une concentration de 80 % à 98 % en poids pendant 20 min à 1 h.

4. Procédé de production pour produire les 2-halo-3,5-difluoroanilines représentées par la formule générale (III) 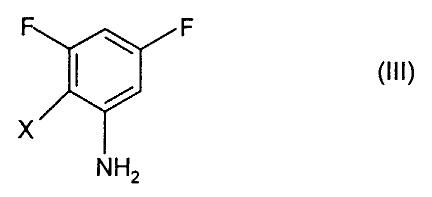 où X est le fluor ou le chlore, comprenant l'étape de :
chauffer le 4-amino-5-halo-2,6-difluoroisophtalyldiamide représenté par la formule générale (II) 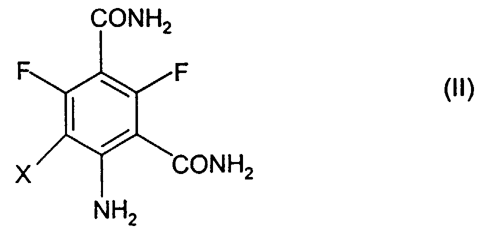 où X a la même signification que celle donnée ci-dessus, en présence d'acide sulfurique à une concentration qui n'est pas inférieure à 30 % en poids à une température de 80°C jusqu'au point d'ébullition du mélange réactionnel.
